Europäisches Patentamt

European Patent Office

Office européen des brevets

(19)

(11) **EP 1 215 993 B1**

(12) **EUROPEAN PATENT SPECIFICATION**

(45) Date of publication and mention
of the grant of the patent:
**22.12.2004 Bulletin 2004/52**

(21) Application number: **00961272.2**

(22) Date of filing: **06.09.2000**

(51) Int Cl.$^7$: **A61B 5/00**

(86) International application number:
**PCT/NO2000/000289**

(87) International publication number:
**WO 2001/017419 (15.03.2001 Gazette 2001/11)**

(54) **SYSTEM FOR PREDICTING THE OUTCOME OF AN IMAGINARY DEFIBRILLATOR SHOCK**

SYSTEM ZUR VORHERSAGE DES RESULTATS EINES IMAGINÄREM
DEFIBRILLATORSCHOCKS

SYSTEME DE REPONSE A UN CHOC IMAGINAIRE DE DEFIBRILLATEUR

(84) Designated Contracting States:
**AT BE CH CY DE DK ES FI FR GB GR IE IT LI LU MC NL PT SE**

(30) Priority: **07.09.1999 NO 994344**

(43) Date of publication of application:
**26.06.2002 Bulletin 2002/26**

(73) Proprietor: **Laerdal Medical AS
4001 Stavanger (NO)**

(72) Inventors:
• **MYKLEBUST, Helge
N-4025 Stavanger (NO)**
• **EFTESTOL, Trygve
N-4033 Forus (NO)**

(74) Representative: **Herbjörnsen, Rut
Albihns Stockholm AB
P.O. Box 5581
114 85 Stockholm (SE)**

(56) References cited:
**US-A- 5 694 943        US-A- 5 724 983
US-A- 5 957 856**

## Description

The present invention regards a system for calculating the probability figure for the outcome of an immediate following defibrillator shock on the basis of properties of the heart measured during sudden cardiac arrest and resuscitation, as stated in the introduction to Claim 1.

Near 40% of all those who suffer sudden cardiac arrest will be able to survive if they receive good, lifesaving treatment immediately. When treatment is delayed, the chances of survival decrease, cf. the article by Holmberg S, Holmberg M: "National register of sudden cardiac arrest outside of hospitals " 1998 [1]. The treatment primarily consists of cardio-pulmonary resuscitation (CPR), which is administered until a defibrillator is in place. Thereafter, the treatment consists of alternating use of a defibrillator and CPR until resuscitation or until an ALS team arrives. (ALS = "Advanced Life Support") The latter also includes medication and securing of the respiratory passages as part of the treatment, cf. ILCOR, "Advisory statements of the International Liaison Committee on Resuscitation. Circulation" 1997;95: 2172-2184 [6]

Scientific papers in recent years point out a number of factors that affect the chances of survival:

Time: The chance of surviving sudden cardiac arrest falls with time from heart failure until the first defibrillator shock is administered.[1]

CPR: The chance of surviving increases when someone administers CPR before the defibrillator arrives. [1]

Quality of Studies show that the quality of the CPR influences the survival.

| CPR: | (Cf. the publications by Wik L, Steen PA, Bircher NG. "Quality of bystander CPR influences outcome after prehospital cardiac arrest. Resuscitation" 1994;28:195-203[2]. Gallagher EJ, Lombardi G, Gennis P. "Effectiveness of bystander CPR and survival following out-of-hospital cardiac arrest". J Am Med Assoc 1995;274:1922-5[3] Van Hoyvegen RJ, Bossaert H. "Quality and efficiency of bystander CPR. Resuscitation" 1993;26:47-52[4]) |
|---|---|
| Timing of CPR and defibrillator treatment: | A study shows that when the duration of sudden cardiac arrest exceeds a number of minutes, the chance of survival will increase if the ambulance personnel first administers a period of CPR before the defibrillator is used. (Cf. Cobb L, et al. "Influence of cardiopulmonary resuscitation in patients with out-of hospital ventricular fibrillation. JAMA", April 7, 1999 - Vol 281, No 13 [5] |

In case of sudden cardiac arrest, the electrical activity in the heart (ECG) will indicate the state of the heart. Today's defibrillators measure and analyse ECG in order to classify the rhythm. If the rhythm is classified as Ventricle Tachyardia (VT) or Ventricle Fibrillation (VF), defibrillator treatment may have an effect. VT is often the precursor of VF. VF will as time goes by and the energy and oxygen reserves of the heart muscle are depleted tend towards Asystole, a rhythm characterised by very little or no electrical activity. The purpose of the defibrillator treatment is to restore the organised electrical activity of the heart and the associated blood pressure and blood circulation. This is often denoted ROSC - "Return of Spontaneous Circulation", and is the first step towards survival.

Only a fraction of the shocks delivered actually result in ROSC. Most shocks today do not give ROSC , cf. the publications Gliner BE et al. "Treatment of out-of hospital cardiac arrest with a Low-Energy Impedance-Compensating Biphasic Waveform Automated External Defibrillator" [7], Sunde K, Eftestøl T, Askenberg C, Steen PA. "Quality evaluation of defibrillation and ALS using the registration module from the defibrillator. Resuscitation" 1999 [14]. In general, it can be said that the chance of ROSC is at its greatest immediately after sudden cardiac arrest, when the heart muscle still possesses energy reserves and oxygen. Many patients achieve ROSC after alternating use of shocks and CPR. The disadvantages of having to give many shocks are several: First of all, no CPR will be given during the shock treatment, a factor that further aggravates the situation for the vital organs, particularly for the brain. Furthermore, it has been shown that the heart muscle is also damaged by the shocks, and that the damage increases with the number of shocks and the amount of energy, cf. the publications Ewy GA, Taren D Bangert J et al. "Comparison of myocardial damage from defibrillator discharges at various dosages. Medical instrumentation" 1980;14:9-12. [16]. For the patient, the ideal would be to be given only one shock, and for this shock to give ROSC.

Thus, for many patients, it is crucial that the administration of CPR be effective, so as to revitalise the heart through supplying a flow of blood through the heart muscle, cf. the publication Michael JR et al. "Mechanism by which augments cerebral and myocardial perfusion during cardiopulmonary resuscitation in dogs. Circulation" 1984;69:

822-835. [17]. This revitalisation can be indicated through ECG measurements, where ECG characteristics such as form, spectral flatness measurements, frequency, amplitude, energy etc. is seen to change back towards their initial values such as they would have been immediately after the heart action was suspended, cf. the publications Eftestøl T, Aase, SO, Husøy JH. "Spectral flatness measure for characterising changes in cardiac arrhythmias." Computers in Cardiology, [15] and Noc M, Weil MH, Gazmuri SS, Biscera J and Tang W. "Ventricular fibrillation voltage as a monitor of the effectiveness of cardiopulmonary resuscitation" in J Lab Clin Med, September 1994 [13]. This revitalisation will increase the probability of the next shock giving ROSC.

Unfortunately, not everyone survives. For many, the reason behind the sudden cardiac arrest is such that resuscitation is impossible. Furthermore, the time factor and the quality of the treatment will also play a part and affect the survival.

Today's resuscitation guidelines describe a protocol that is the same for everyone, regardless of sex. race, how long the heart action has been suspended, whether a member of the public has given CPR etc. The means of resuscitation are primarily CPR and defibrillator treatment, and later also medication administered by lifesavers who have been given special training in this area. Today's protocol is such that if the first three shocks have no effect, CPR is to be given for 1 minute, then three more shocks, and so on. As it takes about one minute to give three shocks, the patient will be without CPR for half of the time.

Literature and other patent applications describe technology, the object of which is to guide the lifesaver in the choice between CPR and defibrillator treatment. Brown et al in US patents no. 5 683 424 and 5 571 142 [10] describe a system that, based on spectral measures in VF, instructs the lifesaver to either give CPR or give a shock. A separate analysis of this method, where the method has been tested on human VF, yields results that show the method to have a low specificity, i.e. that the method will only to a limited degree reduce the number of unnecessary shocks. Noc M, Weil MH, Tang W, Sun S, Pemat A, Bisera J. "Electrocardiographic prediction of the success of cardiac resuscitation" in Crit Care Med 1999 Vol 27 No 4 [12] describe a similar system, based on an animal model, which links the mean amplitude and dominant frequency ofVF to the outcome of the defibrillator shock. Both of these methods aim to advise against defibrillator use as long as the condition of the heart is such that a shock is assumed not to have an effect, and instead use CPR. Both methods define absolute criteria based on a limited number of observations from a defined group of patients or animals.

The object of the present invention is to seek to constantly optimise the treatment through:

a) By means of equipment connected to the patient, measuring the properties of the heart during sudden cardiac arrest, and, based on relevant treatment, knowledge of the patient and knowledge of comparable conditions and outcomes of treatment carried out previously, calculate the probability figure of achieving ROSC - Prosc.
b) Presenting the probability figure or using the probability figure in support of the decision on further treatment.
c) Relaying registrations for each treatment, along with the outcome of each shock, to a centrally located computer, and using this experience to improve the basis for Prosc calculations, so as to improve the confidence of the next calculation.
d) Possibly registering the measured CPR parameters and, together with information regarding medication given, looking at the development of the probability figure for the purpose of identifying effective lifesaving.
e) Possibly providing feedback to the user regarding which CPR parameters and what medications have been identified as effective, or alternatively through using this information in order to instruct the user in effective CPR.

To the extent the word "possibly" is used, this is done in order to take into consideration the fact that there are many categories of users and lifesavers, and based on their professional level and economy, it is appropriate to use the solutions that best serve the purpose.

The object of the invention is to contribute towards giving the patient a treatment that is better suited to the individual, and which gives a greater chance of survival. The use of empirical data makes it possible to allow for differences and the ever-changing patient groups and treatment. To the extent that not all shocks give ROSC, the system attempts to take into consideration factors that affect or may affect the probability of ROSC:

• Properties of the heart that may be observed via the electrodes, and which express the metabolic condition and the pumping action. These properties develop with time during sudden cardiac arrest, but may be partially reversed through the use of medication and CPR.
• Type of defibrillator shock and energy selection produce varying effectiveness.
• Physical conditions. A big patient will receive a lower current density through the heart than a small patient, for the same energy selection.
• Patient information. The system takes into account the fact that there may be a difference between men and women, based on the fact that over 70% of those who suffer sudden cardiac arrest are men. In certain parts of the world, the duration of life has increased, so that the number of elderly who suffer sudden cardiac arrest is increasing. These may very well have received treatment over a period of time, both medications, surgical procedures and aids such as pacemakers, all of which may have an effect on the Prosc.

• Geography, race. The system further takes into consideration the fact that there may be differences based on lifestyle and genetic conditions, just as life expectancy varies greatly depending on geography and race.

The above is provided by means of a system of the type mentioned initially, the characteristics of which are stated in Claim 1. Further characteristics of the invention appear from the remaining, dependent claims

Using Prosc to optimise the treatment may be done in several ways. For advanced users, the most expedient will be to present the parameter graphically versus time, as a trend curve. This will immediately provide a direct indication of the state of the heart, and also indicate the effect of medication and CPR. For groups who are not trained in relating to this type of information, the most appropriate thing will be to provide automatic decision support in the question of whether or not to give CPR, in which way CPR should be given, or whether shocks should be given. The principle of simple decision support may be:

• If Prosc is less than a limit, Grosc, CPR is recommended. Otherwise, a number of sequential defibrillator shocks are recommended.
• CPR is recommended until positive changes in the Prosc level out, but no further than T minutes before a number of defibrillator shocks are recommended.

The following will describe the invention in greater detail, with reference to the drawings, in which:

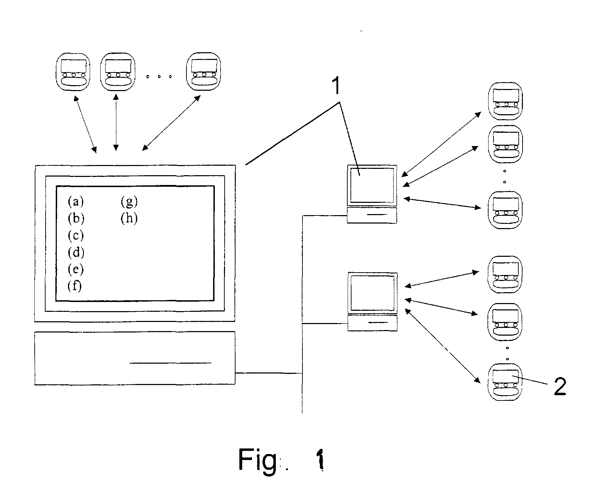

Fig. 1     shows system components consisting of one. alternatively several, computers in a network that communicates with a number of positioned analysis units.
Fig. 2     shows the block diagram for a defibrillator with a built-in analysis unit.
Fig. 3     shows the elementary flow diagram for information.
Fig. 4     shows an apparatus with electrodes connected to the patient's chest, in positions on the chest that are normally used for delivery of a defibrillator shock, as well as for measuring ECG in accordance with standard derivation II.
Fig. 5     shows a flow diagram for development of coefficients to optimal filter.
Fig.6     shows a flow diagram for development of a classification that fulfil the requirement of generality.

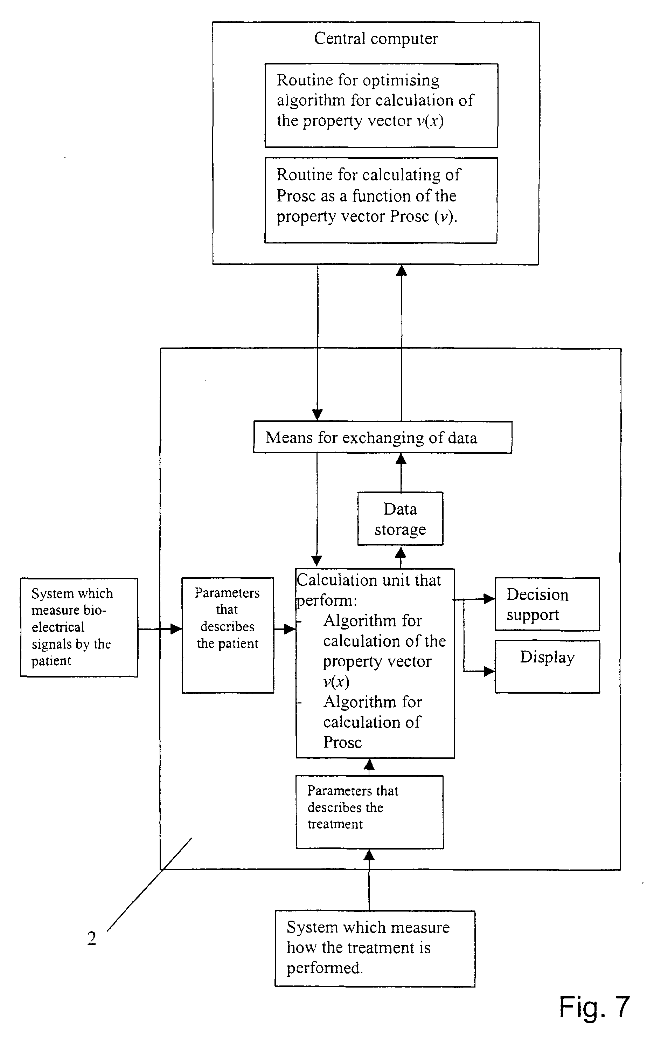

Fig. 7     shows a general block diagram of the invention with focus on the analysis unit.

The system consists of one, alternatively several, computer(s) 1 in a network that can communicate with a number of positioned analysis units 2. These may either be integrated into equipment (U1, U2..) such as defibrillators or ECG monitors, or they may occur in or as a support product used during the resuscitation attempt The analysis units 2 generally operate independently of the computers 1, however after use, the analysis units will deliver field data to the computer 1, and possibly receive adjusted algorithms for calculation of property vector and/or Prosc

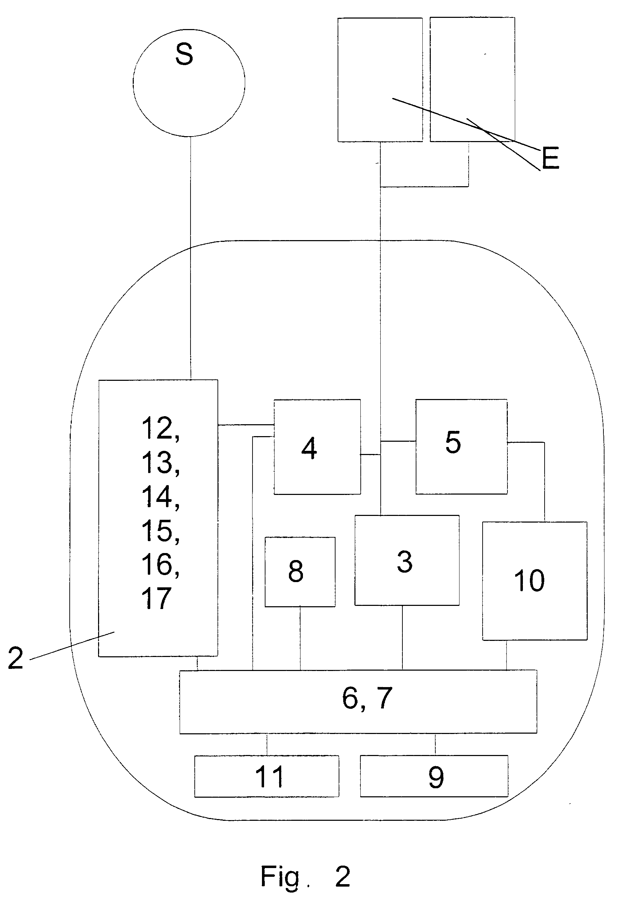

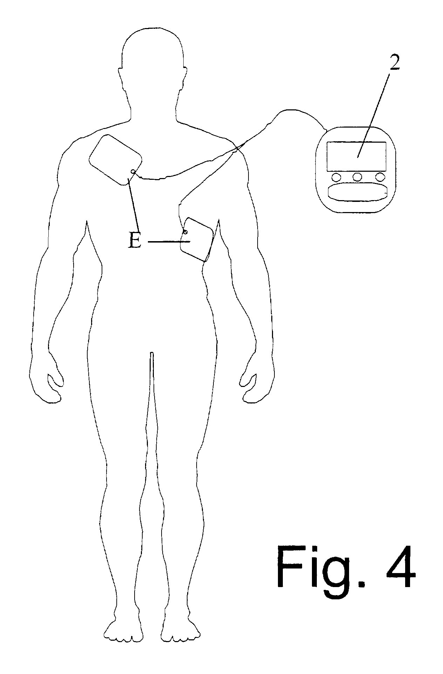

The analysis unit 2 normally has subsystems associated with it, cf. Fig. 2:
Some of these subsystems are standard in equipment such as defibrillators and ECG monitors, and these are as follows:
Reference number 3 denotes a system for measuring ECG, connected to electrodes E. For measuring and digitalize of bio-electrical signals is used said electrodes connected to skin of the patient in accordance with standard derivation II, cf. Fig. 4, which shows the device with electrodes connected to the patient's chest in locations that are normally used for delivering defibrillator shocks, as well as for measuring ECG in accordance with standard derivation II. In this derivation, ECG will essentially express the electrical activity in the longitudinal axis of the heart. Reference number 4 denotes a system for checking the electrode connection to the patient, reference number 5 denotes a device for high voltage and shock delivery, if integrated into a defibrillator, reference number 7 denotes an algorithm for classification of ECG, if integrated into a defibrillator, reference number 7 denotes a microprocessor system and software, reference number 8 denotes computer memory, reference number 9 denotes a user interface, reference number 10 denotes an energy supply, reference number 11 denotes a system for delivery and receipt of data from external equipment.

Subsystems 3-11 are standard equipment in defibrillators and ECG monitors, and will therefore not be described further in this specification.

Analysis unit 2 consists of the following units:
Unit 12 for determining one or more properties of the heart that is combined in a property vector, and based on this calculate the probability of ROSC, Prose, for the patient who is connected up. Possibly also a module 13 for determining the blood flow through the heart, based on the measured impedance and the change of the impedance between the electrodes as a function of the pumping action of the heart and the expansion of the lungs, a possible module 14 for registering CPR parameters from sensors S, a possible module 15 for registering patient specific information, a possible module 16 for registering any medication administered; and a possible module 17 for correlating positive changes in Prosc with information regarding the treatment given, and display or use this information to guide the treatment.

The analysis unit may be realised as a self-contained microprocessor unit, or it may be realised through the microprocessor unit in the equipment.

Each computer consists of the following subsystems:

(a) Hardware, (b) operating system, (c) software and interface for communication in a network (d) database for field data, (e) algorithm for calculation of property vector, including a classification system for predict the outcome of an immediate following defibrillator shock, (f) system for calculation of Prosc, (g) algorithm for correlating changes in Prosc with information regarding patient and treatment, and (h) system for delivery and receipt of data from positioned defibrillators.

With regard to the computers, the subsystems of hardware, operating systems, software and interface are of a generic nature, and will not be described in greater detail.

A more detailed description of the system, first computer 1.

(d) The database for field data consists of a large amount of patient episode data, and contains:

- Patient information: Sex, age, weight, race etc.
- Geographical information
- Information regarding each defibrillator shock: Curve shape, energy, timing versus VF.
- For each shock:
- Preshock ECG
- Preshock CPR data
- Preshock medication data
- Preshock impedance data
- Postshock ECG
- Postshock impedance data
- Annotation of ROSC/Non-ROSC, with outcome rhythm for each shock

(e) The algorithm for calculation of the property vector (v) makes use of mathematical methods in order to characterise the condition of the heart based on a recording of a bio-medical signal (x). The bio-medical signal is preferably ECG, but also other type of signals such as signals derived from the change of the impedance, sound or pattern of the movement may be considered.

The algorithm for calculation of the property vector is hereafter denoted as v(x). v(x) which is used on ECG from empirical data provide us two set of property vectors: An set, V1, containing n1 property vectors where the outcome of the shock is ROSC, and an set, V2, containing n2 property vectors where the outcome of the shock is none-ROSC.

In general, v(x) is defined as an operator that operates on an ECG sequence, x, consisting of $N$ samples, which generates a property vector, v, consisting of $M$ vector elements that ideally takes care of the information in $x$ that separates the group of $x$ that results in ROSC, $X1$, from the group of $x$ that results in non-ROSC, $X2$. The methods of property extraction are innumerable, and the literature describes some of these, which can be roughly divided into time and transform domain methods, where the object is to structure $x$ in a manner that is appropriate for property extraction. Among preferred methods are:

- Optimised digital filters determined by $L$ filter parameters that divides $x$ into $M$ channels. The energy from each of these channels is calculated, so as to make the property vector consist of $M$ elements. These types of filters are described inter alia by T. Randen "Filter and Filter Bank Design for Image Texture Recognition" in a thesis of NTNU, Oktober 1997 where the filters are optimised in order to achieve the best possible recognition of the different textures. For present purpose is the optimised filters found by using a numerical gradient search algorithm (T. Coleman, M. A. Branch and A. Grace, , *Optimization Toolbox for Use with MATLAB,* The Math Works Inc, 1999) to achieve the best possible separation of the ROSC group from the non-ROSC group. Separation ability is measured by the sum of sensitivity (degree of correct recognition of ROSC) and specified (degree of correct recognition of nom-ROSC) By a given iteration in optimisation is this performance measured, and the set of parameters, which define the filters, is adjusted in the direction corresponding the increase in performance. This procedure is repeated until maximum performance is reached.
- Spectral measures that are calculated on the basis of the estimate of the power density spectrum (PSD) of $x$. The PSD can be estimated through use of Fourier transforms. Based on the PSD, characteristics are calculated that express the frequencies at the centre and the maximum points of the PSD. In addition, the flatness and energy of the PSD is also characterised

Examples of other methods for property extraction are:

- wavelet analysis,
- neural networks

The relation between V and X1, V2 and X2 respectively are as following: X1 containing an set of n1 ECG sequences, which, when used on v(x), provides a number of property vectors V1, which all is belonging to the outcome class ROSC (w1). X2 containing an set of n2 ECG sequences, which, when used on v(x), provides a number of property vectors V 1, which all is belonging to the outcome class non-ROSC (w2).

(f) A system for calculation of the Prosc function is based on pattern recognition theory, and forms the second element of the classification system. In this context, the term classes is defined as the collection of measurements of the condition of the heart that corresponds to

- ROSC (*w1*)
- non-ROSC (*w2*)

The property vectors of the two classes are statistically described by

- *P(wi), i*=1,2 , which is the a priori probability of the two classes. I.e. before a measurement is made, the probability of one or the other outcome is known through the respective a priori probabilities.
- *p(v|wi)* are the class specific probability density functions. These express how the measurements within the given classes are distributed. $p(v)$ expresses the compound probability density function for the measurements, and is given by adding up the class specific probability density functions weighted by the associated a posteriori probabilities.
- *P(wi|*$v$*)* are the a posteriori probability functions for the two classes. These functions express the probability of a given measurement belonging to *wi*. Bayes formula expresses *P(wi|*$v$*)* as a function of the above probability functions .
- P(wi|$v$)=P(wi)*p($v$|wi)/(p(w1)+P(w2)*p($v$|w2))
- The sum of the a posteriori probabilities for a given $v$ is always 1.

In the case of a given measurement, $v$, one wishes to determine the class allocation *w1* or *w2*. It has been proven that the expected probability of misclassification is minimised by selecting the *wi* that corresponds to the maximum *P(w*$\lambda$$v$*)*. It is further possible to define (make an estimated choice of) the cost of all types of misclassification, such that the expected risk of a given misclassification is given by the product of the cost and the a posteriori probability of the true class. The expected risk of misclassification can then be minimised by classification is a class corresponding the product with smallest value is selected.

In most cases, the statistics of the property vector are not known. These quantities must then be estimated before Prosc(v) can be produced. The pattern recognition theory describes a multitude of methods for this, which are based on measurements (practice data) that are examples from the various *wi.* Some examples:

- Histogram techniques, which divides the outcome space into hypercubes in which the probabilities within each of these are calculated on the basis of the number of occurrences of the different classes within the given hypercube. This corresponds to the method used herein. In the following is described how statistic quantity is estimated.
  We will start defining the quantities:

  n=total number of observations in the empirical material.
  n1= total number of observations corresponding ROSC outcome.
  n2 = total number of observations corresponding non-ROSC outcome.
  nj1= total number of observations corresponding ROSC outcome within hypercube no. j.
  nj2= total number of observations corresponding non-ROSC outcome within hypercube no. j

  We have n=n1+n2. Estimate for a priori probability will then be ^P(wi)=ni/n, i=1,2.
  The local estimates (within hypercube j) for the class specific probability function will then be

  ^p(v|wi)=nji/ni, I=1,2.

  The local estimates for a posteriori probabilities is calculated in respect of the Bayes formula inserted estimate for a priori probability and the local class specific probability density functions. Se R. J. Schalkoff *Pattern recognition: Statistical, structural and neural approaches.* John Wiley & sons, New York (NY), 1992

$$^\wedge P(wi|v)=nji/(nj1+nj2),\ i=1,2$$

- Radial base functions, in which the probabilities at a given point are calculated on the basis of the contribution from surrounding practice data from the different classes. The contributions decrease with distance.
- Parametric modelling, in which a mean value and dispersion for the different classes are used to produce analytical probability models.
- Neural networks, learning vector quantization and nearest neighbour classification are some other central methods within the pattern recognition theory.

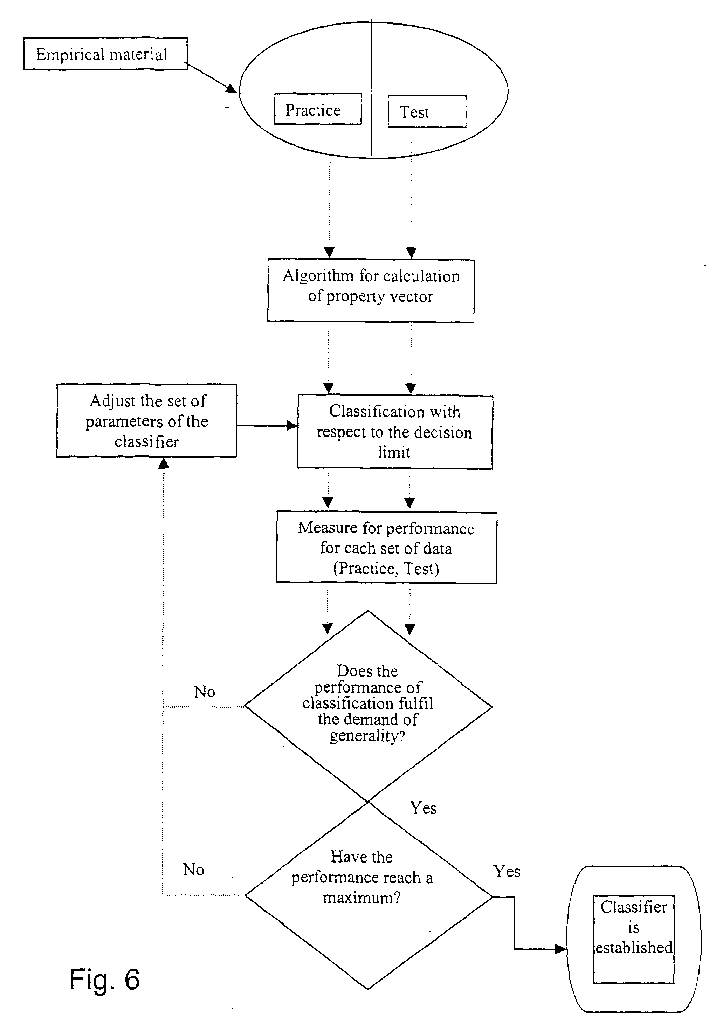

It is important that a given classifier be tested on a set of observations (test set) independently of the practice data (practice set), in order to check that the classifier yields the expected results. The demand is that there is consistency between practice and testing, that the classifier fulfils the requirement of generality. With generality means: By dividing the empirical data in two parts and letting the one part represent a set of data called practice set and the other part represent a set of data called test set, the generality is defined as following: Decision limits used on all of the property vectors in each set of data for classification of the outcome, which provides approximately same performance (the sum of sensitivity and specificity) for both set of data fulfil the requirement of generality. These decision limits occur through an iterative process where the practice set is included in the calculation of the decision limit, see fig. 6.

Those measurements $v$ that correspond to the ROSC outcome belong in $w1$. The probability of a given measurement, $v$, belonging in $w1$ is given by $P(w1|v)$. In other words, this probability function expresses the probability figure *Prosc* of ROSC for a given measurement $v$.

$$Prosc(v)=P(wl|v)$$

As mentioned previously, different property vectors, v, can be calculated by means of a countless number of methods. Which methods and which dimension, M, is suitable for expressing Prosc(v) is assessed on the basis of the expected risk in the case of misclassification for each method. The method that minimises this risk is the most appropriate for expressing Prosc(v). Fig.6 shows a flow diagram for an iterative development of algorithm for calculation of the property vector $v$. Basis for the iterative development is empirical data. As the amount of empirical data increase is this iterative process repeated so that the ability of the property vector to predict outcome classes is increased. The iterative adjustment of the decision limit is also included so that the requirement of generality is fulfilled.

(g) The algorithm for correlating changes in Prosc with information regarding the patient and the treatment is mainly for scientific purposes. The defibrillator to guide the user during lifesaving may later use the results from the correlation.

Prosc($v$) has been provided as described under points (d) and (e). In this analysis, ECG sequences are extracted from the patient material, so that the ECG sequences describe a course of treatment that is as uniform as possible, with the complete patient material seen under one. Examples of such a course of treatment may be

- CPR sequences
- "Hands off" intervals, for instance a period for defibrillator rhythm analysis up to the shock, after a CPR period.

In these ECG sequences, corresponding Prosc($v$) sequences are calculated as described under points (d) and (e). Consequently, the change in Prosc($v$) , DProsc, is calculated for each sequence. DProsc is grouped on the basis of those treatment characteristics that are of interest with regard to the effect of the treatment. As an example, one can group DProsc with regard to the following treatment characteristics, singly or in combination:

- Different compression frequencies, compression depths, duration of chest compression
- Degree of ventilation
- Medication
- Physiological measurements such as blood flow measurements, blood pressure etc.

Where significant differences in DProsc occur for dissimilar treatment conditions, this information may be used to identify advantageous treatment methods. This information may be utilised through the person giving the treatment being given feedback regarding good and poor treatment.

(h) A system for delivering and receiving data from positioned analysis units. Here, no special requirements apply. The exchange of data can take place directly through use of memory modules such as PCMCIA, cordlessly by means of IR or RF communication, via networks such as the Internet, or by a direct connection between communication ports in the equipment and the computer. The most practical method these days is to have the analysis unit 2 communicate directly with the computer 1 via a local PC that it can communicate with, and to have the local computer pass the data on via the Internet.

Detailed description of the analysis unit 2.

System 12, algorithm v(x) for calculating the property vector (v) and algorithm for calculating the probability of ROSC, Prosc, for ECG from the patient who is connected up.

- v(x)is a set with calculation, which combined form a property vector. The calculation is a set of energy calculation within a determined frequency band (optimised filter) or a set of parameters diverted from effect density spectrum or a combination of this.
- The algorithm for calculation of Prose is typical a matrix where the number of dimension is consistent with the number of dimensions within the property vector, where each matrix element is containing a numerical value for pROSC. The numerical value is downloaded from the computer 1. The matrix is provided in that there for each ECG sequens within the empirical material is calculated a property vector. Property vectors that are approximately identical are grouped thereafter together in a number of sets, where each set is assigned a matrix element. For an set, j, the occurrences of ROSC is counted, nj 1, towards the occurrences of non-ROSC, nj2. The ratio between the numbers of {ROSC} towards the number of {ROSC+non-ROSC} define the numerical value of pROSC for the set j which is nj1/(nj1+nj2). This correspond the local estimates for a posteriori probability for the class w1 as described by use of histogram technique. The numerical value for each set is then stored in that matrix element that correspond to the elements of the property vector.

The module 13 for calculating blood flow through the heart based on the measured impedance and the change of the impedance between the electrodes as a function of the pumping action of the heart and the expansion of the lungs:

- The value of the measured impedance, Zo, measured by means of an approximately constant alternating current, informs the analysis unit 1 of the impedance between the electrodes, and can be used to replace system 4.
- The impedance change between the electrodes will be proportional to the change in the set of air in the lungs plus the working volume of the heart. The change due to air dominates. By looking at the signal between two ventilations, or by first filtering out the ventilation, it will be possible to estimate the working volume on the basis of the formula

$$\Delta V = \frac{\Delta Z \cdot \rho L^2}{Zo^2}$$

This formula is universally known, and is used within Impedance Cardiography. $\Delta Z$ is the impedance change, $\rho$ is the resistivity of the blood, $L$ is the distance between the electrodes, and $Zo$ is the numerical value of the impedance. A simplification of this formula is preferable:

$$\Delta V = \frac{\Delta Z k}{Zo^2}$$

Here, k is a constant. This measurement will indicate to what degree the blood is flowing, and will contribute towards characterising the condition of the heart in VF/VT, and will furthermore indicate ROSC in case of a successful defibrillator shock.

Module 14 for measuring and registering CPR parameters.

Relevant CPR parameters are:

- Inflation time and inflation volume are measured by looking at the impedance change between the electrodes. This change is several times greater than the change that takes place as a function of the blood stream from the heart, and is proportional to the amount of air in the lungs. The principle is known from other diagnostic equipment.
- Compression rate and compression proportion (the ratio between compression time and relaxation time) during chest compression may be measured by looking at the impedance change between the electrodes, or by placing a sensor at the compression point on the patient's chest. This sensor can contain a pressure switch, or a dynamometer or an accelerometer.
- Compression depth is calculated on the basis of signals from an accelerometer placed at the compression point.
- Time between inflation and chest compression, and time between chest compression and inflation.
- Proportion of CPR relative to the total treatment time

- Amount of compression, the sum of the product between the duration and depth of the compression.

Module 15 for indicating patient specific information.
This information can be passed to the analysis unit 1 e.g. by dedicated push buttons, or it may come in from an external source such as a patient database or a patient journal on a PC / handheld computer. Relevant information is:

- Geographical area
- Age
- Sex
- Weight
- Race

Module 16 for indicating medication and dosage given.
This information can be passed on to the analysis unit 1 through dedicated push buttons, from a patient journal on a PC or from other devices that log the use of medication.
Relevant medicines are

- Epinephrine
- Lidocaine
- Bretylium
- Magnesium sulphate
- Procainamide
- Vasopressins
- Thrombolysis medication
- and so on.

Module 17 for correlating positive changes in Prosc with information regarding the treatment given, and displaying or using this information to guide the treatment.

- The system identifies and registers a period of Prosc with positive change. At the same time, the system identifies and registers the average of each CPR parameter measured for a period of time prior to the change and during the change, and if applicable, what medication was given during the same period.
- If a new period of improved positive change occurs, the identification of CPR parameters and medication is repeated.
- This information can be displayed on the defibrillator screen, or it may be used to produce voice messages that guide the user to deliver CPR with parameters that are in accordance with the registration.
- This information will also be of great importance to research, with a view to optimising the guidelines for CPR treatment and training.

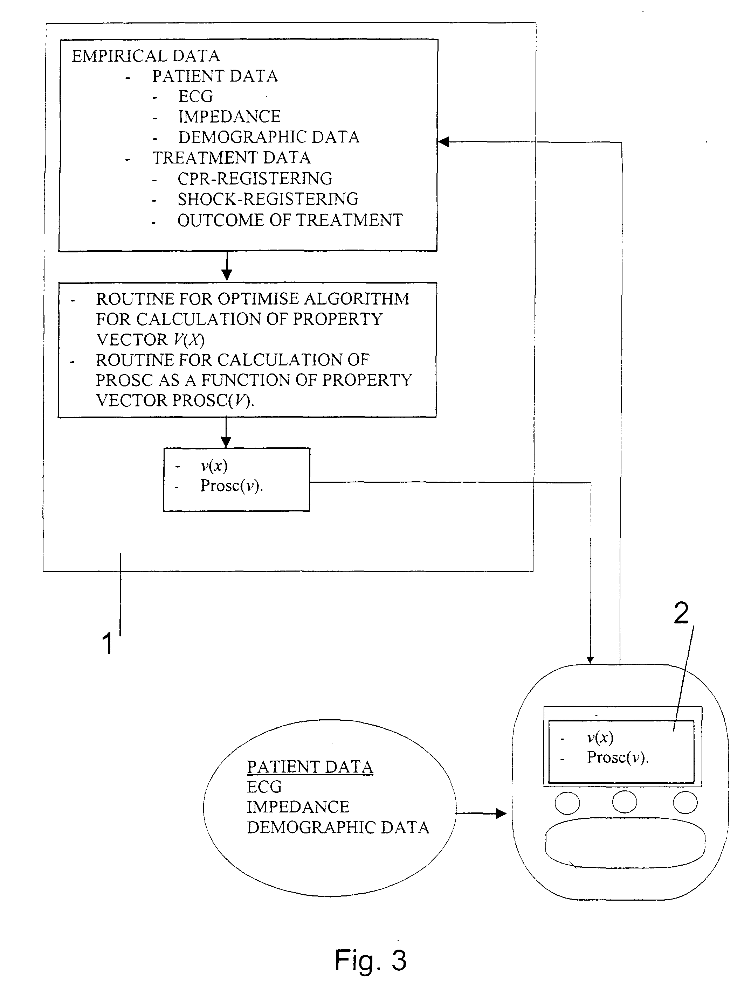

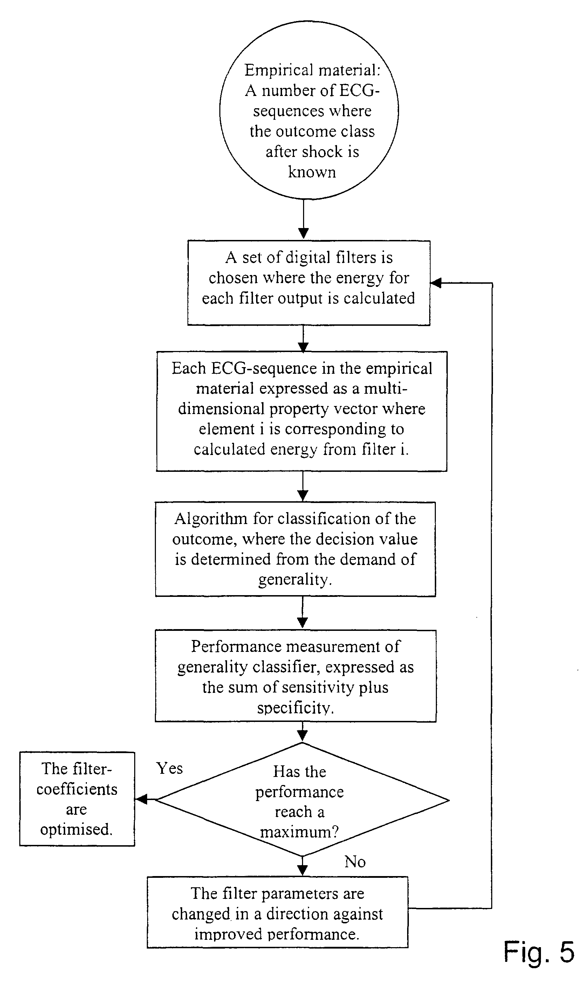

To summarise, the invention can be described with reference to Figure 3, which schematically shows the flow of information between the central computer 1 and the analysis unit 2 in the positioned equipment in an application of the invention.
The computer 1 contains empirical data from previous resuscitation attempts, where the outcome of the resuscitation attempt is known. The main ingredient in the empirical basis is the ECG and the associated outcome after a shock (ROSC/non-ROSC). Additional empirical data impart nuances to the relationship between outcome, treatment and patient specific factors.
An algorithm $v(x)$ for calculating of a property vector $v$ is developed based on empirical material. This algorithm is adapted so that the ability of the property vector to predict the outcome (ROSC/non-ROSC) is optimised within the demand of generality.
A property vector $v$ is thus characterised by that it represents ECG segments that is succeeded by ROSC after shock characteristically different from ECG segments that is succeeded by non-ROSC after shock. Such property vector is provided through an iterative process where the parameters for each iterative step is being adjusted until the best performances are reached, see fig. 5.
By grouping the property vectors in the empirical data set that are approximately identical and adding up the number of cases of ROSC and the number of cases of non-ROSC for each of these sets, then the probability figure Prosc for ROSC is expressed statistically relative to the value of an in-coming property vector which represents the ECG segment Prosc is desired to be calculated for.
The property vector of a ECG segment consist of a number of element which together characterize the ECG segment with a view to the outcome after a shock. Let the property vector consist of (n+p) elements that together describes one determined ECG segment. Then the property vector will be n energy calculations of the output signal of n different digital filters, where the ECG segment constitute the input signal plus p parameters that express characterizing features by the power density spectrum of the ECG segment. Preferred characterizing features is: Frequency by the centre of gravity, frequency by the maximum point, spectral flatness measurements, and spectral energy. These parameters will to a limited degree overlap each other in view of information contents. In order to reduce the set of data without to reduce the number of information may for example be used principal component analysis. By such a method may a big set of data, which is described of (n+p) elements in each vector be reduced to one set of data where each vector only have m elements; m<(n+p).

A practical way of expressing this statistical interrelationship is through a Prosc function, which is a substitute for all the empirical data, but which mathematically expresses the same relationship between the property vector and Prosc.

This function is entered into the programme code of the analysis unit, so that when this receives a segment of ECG, the analysis unit will first perform the same calculation of the property vector as that performed by the computer, and then use the property vector as input to the Prosc function in order to calculate the probability figure of an immediate following defibrillator shock giving ROSC.

The analysis unit may furthermore impart nuances to, and thereby provide a more accurate probability figure, by also utilising knowledge of the treatment and the patient, seen in relation to empirical data.

The ever-changing forms of treatment and patient characteristics necessitate a continuous update of the empirical basis. This is achieved by each analysis unit passing on its experience regarding each shock to the central computer, where the central computer repeats the grouping of the property vectors, recalculates the Prosc function and passes the result back to the analysis unit.

## Claims

1.  A system for calculating probability figures for the outcome of an immediate following defibrillator shock resulting in return of spontaneous circulation (ROSC)
    wherein
    an analysis unit is connected with a module, which is measuring bio-electrical signals from electrodes connected to a patient,
    the analysis unit is provided to organise the bio-electrical signals continuous into segments,
    the analysis unit is provided for each segments to calculate a combination parameter that characterise the condition of the heart
    the analysis unit is provided to by means of comparing for each combination of parameters to find a corresponding combination of parameters from earlier made defibrillator treatments, where there for each combination of parameters is assigned a probability figure, where the probability figure expresses the number of defibrillator shocks that results in ROSC relative the total number of defibrillator shock for each combination of parameters, and
    the analysis unit has an output for the probability figure.

2.  A system according to Claim 1, wherein
    the bio-electrical signals is ECG signals.

3.  A system according to Claim. 1-2 wherein
    the analysis unit (2) is provided to calculate the probability figure by means of an algorithm.

4.  A system according to Claims 1-3 , wherein the algorithm for calculation the probability figure is a table look up in a m-dimensional table, where there for each table element is stored a numerical value for the probability figure, the table look up is determined from the value of a m-dimensional vector, the value of the m-dimensional vector is diverted from the calculation of the energy of respective m different signal sequences that is represented on the output of m different digital filters, where the signal on the input of each digital filter is the segment of the ECG signal.

5.  A system according to Claims 4, wherein
    the value of the m-dimensional vector is diverted from calculation of flatness, energy, frequency by the centre of gravity and frequency by the maximum point of a power density spectrum, where the power density spectrum is diverted from the ECG signal segment.

6.  A system according to Claims 1-5, wherein the calculation unit is connected to a data storage, the calculation unit is storing for each treatment parameters which describe the patient and parameters which describes the treatment, the calculation unit is connected to means for exchange of data, the exchange of data occur on a regular basis towards a central computer, where the calculation unit receives optimised algorithm for calculation of the probability figure, and the computer receives information that is stored en the data storage.

**7.** A system according to Claims 1-6, wherein
there is provided an optimised algorithm by first establishing of an updated set of empirical data consisting of information from a number of new patient treatments together with information from a number of earlier performed patient treatments, which all contain sequences of ECG where the outcome after shock are known; the optimised algorithm occur by iterative search after filter coefficients by m digital filters, where the filter coefficients are adjusted iterative in view of performance of a classification routine, where again the classification routine is adjusted iterative in view of performance and generality, where the performance is defined as the sum of sensitivity and specificity for classification of each of the ECG sequences to outcome classes ROSC and non-ROSC respectively, where the real outcome of shock is known for each ECG sequence, generality is fulfilled as the classification routine has the same performance for a arbitrary composite half of empirical material as for the rest of the empirical material, where measurement of generality and performance is provided in that each ECG sequence in the empirical material is expressed as a m-dimensional vector calculated from energy at the output of m digital filters, where the classification routine classifies each in-dimensional vector to one of the outcome classes ROSC, non-ROSC respectively, where the performance is measured as the sum of sensitivity and specificity of the classification routine, where a arbitrary composite half of empirical material has the same performance as the rest of the empirical material, the optimised algorithm for calculation of the probability figure consist of a matrix having m matrix elements, where each matrix element express a probability figure, where the probability figure for each matrix element is provided by grouping ECG-sequences which is expressed by approximately identical m-dimensional vectors, where the occurrence of ECG which resulted in ROSC by shock plus the sum of occurrence of ECG which resulted in non-ROSC by shock constitute the probability figure for the matrix element, the m-dimensional matrix together with the filter coefficient constitute the optimised algorithm for calculation of the probability figure.

**8.** A system according to Claims 1 wherein
the output of the analysis unit is connected to a receiver in the shape of a display unit.

**9.** A system according to Claim 8, wherein
the receiver is a defibrillator.

**10.** A system according to Claims 1, wherein the receiver of the probability figure is an algorithm for decision support for the choice of treatment.

**11.** A system according to Claims 1, wherein
the analysis unit (2) identifies periods of positive change in the probability figure together with parameters that characterise the treatment, and passes on the numerical value of the positive change in the probability figure, together with the mean value of each treatment parameter over the period, to a receiver.

**12.** A system according to Claims 1, wherein
the receiver of the numerical value of the positive change in the probability figure, together with the mean value of each treatment parameter over the period, is a display unit.

**13.** A system according to Claims 1, wherein
the receiver of the numerical value of the positive change in the probability figure, together with the mean value of each treatment parameter over the period, is an algorithm for decision support for the choice of treatment.

**14.** A system according to Claims 1-13, wherein
a device for indicating patient specific information and/or specific information regarding the treatment is connected to the analysis unit (2).

**Patentansprüche**

**1.** System zum Berechnen von Wahrscheinlichkeitszahlen für das Resultat eines unmittelbar darauffolgenden Defibrillatorschocks, der zum Wiedereinsetzen des spontanen Kreislaufs (Return of Spontaneous Circulation-ROSC) führt,

wobei

ein Analysegerät mit einem Modul verbunden ist, das über Elektroden, die an einen Patienten angeschlossen sind, bioelektrische Signale misst,

das Analysegerät dafür konfiguriert ist, die bioelektrischen Signale kontinuierlich zu Segmenten zu ordnen,

das Analysegerät dafür konfiguriert ist, aus den einzelnen Segmenten einen Kombinationsparameter zu berechnen, der den Herzzustand kennzeichnet,

das Analysegerät dafür konfiguriert ist, anhand eines Vergleichs jeder Kombination von Parametern eine entsprechende Kombination von Parametern zu finden, die aus früheren Defibrillatorbehandlungen gewonnen wurden, wobei jeder Kombination von Parametern eine Wahrscheinlichkeitszahl zugewiesen wird, wobei die Wahrscheinlichkeitszahl die Anzahl an Defibrillatorschocks, die zu einem Wiedereinsetzen des spontanen Kreislaufs führen, im Verhältnis zur Gesamtzahl an Defibrillatorschocks für jede Kombination von Parametern ausdrückt, und

das Analysegerät über eine Ausgabemöglichkeit für die Wahrscheinlichkeitszahl verfügt.

2. System nach Anspruch 1, wobei es sich bei den bioelektrischen Signalen um ECG-Signale handelt.

3. System nach Anspruch 1 und 2, wobei das Analysegerät (2) dafür konfiguriert ist, die Wahrscheinlichkeitszahl anhand eines Algorithmus' zu berechnen.

4. System nach den Ansprüchen 1 bis 3, wobei der Algorithmus zum Berechnen der Wahrscheinlichkeitszahl aus dem Nachschlagen in einer m-dimensionalen Tabelle besteht, wobei für jedes Tabellenelement ein numerischer Wert für die Wahrscheinlichkeitszahl gespeichert ist, wobei das Nachschlagen in der Tabelle aus dem Wert eines m-dimensionalen Vektors bestimmt wird, und wobei der Wert des m-dimensionalen Vektors aus der Berechnung der Energie von jeweiligen m verschiedenen Signalsequenzen, die am Ausgang von m verschiedenen digitalen Filtern dargestellt ist, hergeleitet wird, wobei das Signal am Eingang eines jeden digitalen Filters das Segment des ECG-Signals ist.

5. System nach Anspruch 4, wobei der Wert des m-dimensionalen Vektors aus der Berechnung der Flachheit, der Energie, der Frequenz am Schwerpunkt und der Frequenz am höchsten Punkt eines Leistungsdichtespektrums hergeleitet wird, wobei das Leistungsdichtespektrum aus dem ECG-Signalsegment hergeleitet wird.

6. System nach den Ansprüchen 1 bis 5, wobei die Berechnungseinheit mit einem Datenspeicher verbunden ist, wobei die Berechnungseinheit für jede Behandlung Parameter, welche den Patienten beschreiben, und Parameter, welche die Behandlung beschreiben, speichert, wobei die Berechnungseinheit mit einem Datenaustauschmittel verbunden ist, wobei der Datenaustausch regelmäßig an einen Zentralcomputer erfolgt, wobei die Berechnungseinheit einen optimierten Algorithmus zur Berechnung der Wahrscheinlichkeitszahl erhält und der Computer Informationen erhält, die in dem Datenspeicher gespeichert werden.

7. System nach den Ansprüchen 1 bis 6, wobei ein optimierter Algorithmus bereitgestellt wird, indem zuerst eine aktualisierte Menge empirischer Daten zusammengestellt wird, die aus Informationen von einer Anzahl neuer Patientenbehandlungen sowie aus Informationen von einer Anzahl früherer Patientenbehandlungen bestehen, die allesamt ECG-Sequenzen enthalten und bei denen das Resultat nach dem Schock bekannt ist, wobei der optimierte Algorithmus durch iterative Suche nach Filterkoeffizienten durch m digitale Filter abläuft, wobei die Filterkoeffizienten iterativ im Hinblick auf die Leistung einer Klassifizierungsroutine eingestellt werden, wobei wiederum die Klassifizierungsroutine iterativ im Hinblick auf die Leistung und Allgemeingültigkeit eingestellt wird, wobei "Leistung" definiert ist als die Summe aus Empfindlichkeit und Spezifität für die Klassifizierung jeder ECG-Sequenz in die Resultatsklassen "ROSC" bzw. "Nicht-ROSC", wobei das tatsächliche Resultat des Schocks für jede ECG-Sequenz bekannt ist, wobei die Bedingung der Allgemeingültigkeit erfüllt ist, wenn die Klassifizierungsroutine für eine willkürlich zusammengesetzte Hälfte aus empirischem Material die gleiche Leistung wie für den übrigen Teil des empirischen Materials aufweist, wobei die Messung von Allgemeingültigkeit und Leistung in der Weise erfolgt, dass jede ECG-Sequenz in dem empirischen Material als ein m-dimensionaler Vektor ausgedrückt wird, der anhand der Energie am Ausgang von m digitalen Filtern berechnet wird, wobei die Klassifizierungsroutine jeden m-dimensionalen Vektor in eine der Resultatsklassen "ROSC" bzw. "Nicht-ROSC" klassifiziert, wobei die Leistung als die Summe aus Empfindlichkeit und Spezifität der Klassifizierungsroutine gemessen wird, wobei eine willkürlich zusammengesetzte Hälfte aus empirischem Material die gleiche Leistung wie der übrige Teil des empirischen Materials aufweist, wobei der optimierte Algorithmus zum Berechnen der Wahrscheinlichkeitszahl aus einer Matrix mit m Matrixelementen besteht, wobei jedes Matrixelement eine Wahrscheinlichkeitszahl ausdrückt, wobei die Wahrscheinlichkeitszahl für jedes Matrixelement durch Gruppieren von ECG-Sequenzen erzeugt wird, was durch ungefähr identische m-dimensionale Vektoren ausgedrückt wird, wobei das Auftreten von ECGs, die zu ROSC durch Schock geführt haben, und die Summe des Auftretens von ECGs, die zu Nicht-ROSC durch Schock geführt haben, die Wahrscheinlichkeitszahl für das Matrixelement bilden, wobei die m-dimensionale Matrix zusammen mit dem Filterkoeffizienten den optimierten Algorithmus für die Berechnung der Wahrscheinlichkeitszahl bildet.

8. System nach Anspruch 1, wobei der Ausgang des Analysegerätes mit einem Empfänger in Form eines Anzeigegerätes verbunden ist.

9. System nach Anspruch 8, wobei der Empfänger ein Defibrillator ist.

10. System nach Anspruch 1, wobei der Empfänger der Wahrscheinlichkeitszahl ein Algorithmus für die Entscheidungshilfe zur Wahl der Behandlung ist.

11. System nach Anspruch 1, wobei das Analysegerät (2) Zeiträume einer positiven Veränderung der Wahrscheinlichkeitszahl zusammen mit Parametern, welche die Behandlung kennzeichnen, erkennt, und den numerischen Wert der positiven Veränderung der Wahrscheinlichkeitszahl zusammen mit dem mittleren Wert jedes Behandlungsparameters über den Zeitraum an einen Empfänger weiterleitet.

12. System nach Anspruch 1, wobei der Empfänger des numerischen Wertes der positiven Veränderung der Wahrscheinlichkeitszahl zusammen mit dem mittleren Wert jedes Behandlungsparameters über den Zeitraum ein Anzeigegerät ist.

13. System nach Anspruch 1, wobei der Empfänger des numerischen Wertes der positiven Veränderung der Wahrscheinlichkeitszahl zusammen mit dem mittleren Wert jedes Behandlungsparameters über den Zeitraum ein Algorithmus für die Entscheidungshilfe zur Wahl der Behandlung ist.

14. System nach den Ansprüchen 1 bis 13, wobei eine Vorrichtung zum Anzeigen patientenspezifischer Informationen und/oder behandlungsspezifischer Informationen an das Analysegerät (2) angeschlossen ist.

**Revendications**

1. Système permettant de calculer la probabilité qu'un choc de défibrillateur immédiat résulte en un retour à la circulation spontanée (ROSC), dans lequel :

   une unité d'analyse est reliée à un module, qui mesure des signaux bio-électriques provenant d'électrodes reliées à un patient,
   l'unité d'analyse est destinée à organiser les signaux bio-électriques continus en segments,
   l'unité d'analyse est destinée, pour chaque segment, à calculer un paramètre de combinaison qui caractérise l'état du coeur,
   l'unité d'analyse est destinée, par une comparaison de chaque combinaison de paramètres, à trouver une combinaison de paramètres correspondante à partir de traitements au défibrillateur effectués précédemment, où une probabilité est attribuée pour chaque combinaison de paramètres, cette probabilité exprimant le nombre de chocs de défibrillateur qui se traduit par un ROSC par rapport au nombre total de chocs de défibrillateur pour chaque combinaison de paramètres, et
   l'unité d'analyse a une sortie pour la probabilité.

2. Système selon la revendication 1, dans lequel les signaux bio-électriques sont des signaux ECG.

3. Système selon les revendications 1 et 2, dans lequel l'unité d'analyse (2) est prévue pour calculer la probabilité au moyen d'un algorithme.

4. Système selon les revendications 1 à 3, dans lequel l'algorithme pour le calcul de la probabilité est une consultation de table à m dimensions, où pour chaque élément de la table, une valeur numérique est stockée pour la probabilité, la consultation de la table est déterminée à partir de la valeur d'un vecteur à m dimensions, la valeur du vecteur à m dimensions provient du calcul de l'énergie de m séquences de signal différentes qui est représentée à la sortie de m filtres numériques différents, où le signal en entrée de chaque filtre numérique est le segment du signal ECG.

**5.** Système selon la revendication 4, dans lequel la valeur du vecteur à m dimensions provient du calcul de la planéité, de l'énergie, de la fréquence par le centre de gravité et la fréquence par le point maximum d'un spectre de densité de puissance, où le spectre de densité de puissance est obtenu à partir du segment de signal ECG.

**6.** Système selon les revendications 1 à 5, dans lequel l'unité de calcul est reliée à un support de données, l'unité de calcul stocke pour chaque traitement des paramètres qui décrivent le patient et des paramètres qui décrivent le traitement, l'unité de calcul est reliée à un moyen d'échange de données, l'échange de données se produit de façon régulière vers un ordinateur central, où l'unité de calcul reçoit un algorithme optimisé pour le calcul de la probabilité, et l'ordinateur reçoit des informations qui sont stockées sur le support de données.

**7.** Système selon les revendications 1 à 6, dans lequel on fournit un algorithme optimisé en établissant d'abord un ensemble mis à jour de données empiriques constituées d'informations provenant d'un certain nombre de nouveaux traitements de patient ainsi que d'informations provenant d'un certain nombre de traitements du patient effectués précédemment, qui contiennent tous des séquences d'ECG où le résultat après le choc est connu, l'algorithme optimisé se déroule par une recherche itérative des coefficients de filtre par m filtres numériques, les coefficients de filtre étant ajustés de façon itérative au vu de la performance d'une routine de classification, la routine de classification elle-même étant ajustée de façon itérative au vu de la performance et de la généralité, la performance étant définie comme la somme de la sensibilité et de la spécificité pour la classification de chacune des séquences ECG pour donner respectivement des classes ROSC et non ROSC, où le résultat réel du choc est connu pour chaque séquence ECG, la généralité est satisfaite lorsque la routine de classification a la même performance pour une moitié composite arbitraire de matériau empirique que pour le reste du matériau empirique, la mesure de la généralité et de la performance étant fournie dans la mesure où chaque séquence ECG du matériau empirique est exprimée sous la forme d'un vecteur à m dimensions calculé à partir de l'énergie à la sortie de m filtres numériques, où la routine de classification classe chaque vecteur à m dimensions dans l'une des classes. de résultat ROSC et non ROSC, respectivement, où la performance est mesurée comme la somme de la sensibilité et de la spécificité de la routine de classification, une moitié composite arbitraire de matériau empirique ayant la même performance que le reste du matériau empirique, l'algorithme optimisé pour le calcul de la probabilité consiste en une matrice à m éléments, où chaque élément de matrice exprime une probabilité, la probabilité de chaque élément de matrice étant fournie en groupant les séquences ECG qui sont exprimées par des vecteurs à m dimensions à peu près identiques, où l'occurrence d'ECG qui ont donné un ROSC par choc plus la somme des occurrences d'ECG qui ont donné un non ROSC par choc constitue la probabilité pour l'élément de matrice, la matrice à m dimensions, avec le coefficient de filtre, constituant l'algorithme optimisé pour le calcul de la probabilité.

**8.** Système selon la revendication 1, dans lequel la sortie de l'unité d'analyse est reliée à un récepteur qui se présente sous la forme d'une unité d'affichage.

**9.** Système selon la revendication 8, dans lequel le récepteur est un défibrillateur.

**10.** Système selon la revendication 1, dans lequel le récepteur de la probabilité est un algorithme d'aide à la décision pour le choix du traitement.

**11.** Système selon la revendication 1, dans lequel l'unité d'analyse (2) identifie les périodes de variation positive de la probabilité ainsi que les paramètres qui caractérisent le traitement, et transmet la valeur numérique de la variation positive de la probabilité, ainsi que la valeur moyenne de chaque paramètre de traitement sur la période, à un récepteur.

**12.** Système selon la revendication 1, dans lequel le récepteur de la valeur numérique de la variation positive de la probabilité, et de la valeur moyenne de chaque paramètre de traitement sur la période, est une unité d'affichage.

**13.** Système selon la revendication 1, dans lequel le récepteur de la valeur numérique de la variation positive de la probabilité, et de la valeur moyenne de chaque paramètre de traitement sur la période, est un algorithme d'aide à la décision pour le choix du traitement.

**14.** Système selon les revendications 1 à 13, dans lequel un dispositif servant à indiquer des informations spécifiques du patient et/ou des informations spécifiques concernant le traitement est relié à l'unité d'analyse (2).

(a)    (g)
(b)    (h)
(c)
(d)
(e)
(f)

1

2

Fig. 1

Fig. 2

EMPIRICAL DATA
- PATIENT DATA
  - ECG
  - IMPEDANCE
  - DEMOGRAPHIC DATA
- TREATMENT DATA
  - CPR-REGISTERING
  - SHOCK-REGISTERING
  - OUTCOME OF TREATMENT

- ROUTINE FOR OPTIMISE ALGORITHM FOR CALCULATION OF PROPERTY VECTOR $V(X)$
- ROUTINE FOR CALCULATION OF PROSC AS A FUNCTION OF PROPERTY VECTOR PROSC($V$).

- $v(x)$
- Prosc($v$).

1

2

PATIENT DATA
ECG
IMPEDANCE
DEMOGRAPHIC DATA

- $v(x)$
- Prosc($v$).

**Fig. 3**

Fig. 4

Empirical material:
A number of ECG-
sequences where
the outcome class
after shock is
known

A set of digital filters is
chosen where the energy for
each filter output is calculated

Each ECG-sequence in the empirical
material expressed as a multi-
dimensional property vector where
element i is corresponding to
calculated energy from filter i.

Algorithm for classification of the
outcome, where the decision value
is determined from the demand of
generality.

Performance measurement of
generality classifier, expressed as
the sum of sensitivity plus
specificity.

The filter-
coefficients
are
optimised.

Yes

Has the
performance
reach a
maximum?

No

The filter parameters are
changed in a direction against
improved performance.

Fig. 5

Empirical material

Practice    Test

Algorithm for calculation
of property vector

Adjust the set of
parameters of the
classifier

Classification with
respect to the decision
limit

Measure for performance
for each set of data
(Practice, Test)

No

Does the
performance of
classification fulfil
the demand of
generality?

Yes

No

Have the
performance reach a
maximum?

Yes

Classifier
is
established

Fig. 6

Central computer

Routine for optimising
algorithm for calculation of
the property vector $v(x)$

Routine for calculating of
Prosc as a function of the
property vector Prosc $(v)$.

Means for exchanging of data

Data
storage

System which
measure bio-
electrical
signals by the
patient

Parameters
that
describes
the patient

Calculation unit that
perform:
- Algorithm for
  calculation of the
  property vector
  $v(x)$
- Algorithm for
  calculation of
  Prosc

Decision
support

Display

Parameters that
describes the
treatment

System which measure
how the treatment is
performed.

2

Fig. 7